# EUROPÄISCHE PATENTSCHRIFT

(11) **EP 0 959 109 B1**
(45) Veröffentlichungstag und Bekanntmachung des Hinweises auf die Patenterteilung: **09.07.2003**
(21) Anmeldenummer: 99107152.3
(22) Anmeldetag: 13.04.1999
(51) Int. Cl.: C09C 1/00

(54) **Goniochromatische Glanzpigmente auf Basis in einer reduzierenden Atmosphäre erhitzter, titandioxidbeschichteter silikatischer Plättchen**
Goniochromatic gloss pigments based on silicate pigments coated with titaniumdioxide, which are heat treated in a reducing atmosphere
Pigments brillants goniochromatiques à base de pigments silicates recouvert de dioxyde de titane, qui sont chauffés sous une atmosphère réductrice

(30) Priorität: 16.05.1998 DE 19822046
(43) Veröffentlichungstag der Anmeldung: 24.11.1999
(73) Patentinhaber: BASF AKTIENGESELLSCHAFT, 67056 Ludwigshafen (DE)
(72) Erfinder: Schmid, Raimund, Dr., 67435 Neustadt (DE); Seeger, Oliver, Dr., 68163 Mannheim (DE); Mronga, Norbert, Dr., 69221 Dossenheim (DE)

(56) Entgegenhaltungen:
- EP-A- 0 753 545
- EP-A- 0 832 943
- US-A- 5 607 504

## Beschreibung

Die vorliegende Erfindung betrifft neue goniochromatische Glanzpigmente auf der Basis von in einer reduzierenden Atmosphäre erhitzten, titandioxidbeschichteten silikatischen Plättchen, die mindestens ein Schichtpaket aus
A) einer farblosen Beschichtung mit einem Brechungsindex n ≤ 1,8 und
B) einer farblosen Beschichtung mit einem Brechungsindex n ≥ 2,0 aufweisen.

Weiterhin betrifft die Erfindung die Herstellung dieser Glanzpigmente sowie ihre Verwendung zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen der Technik eingesetzt, beispielsweise in Automobillacken, in der dekorativen Beschichtung, der Kunststoffeinfärbung, in Anstrichfarben, Druckfarben, insbesondere Sicherheitsdruckfarben, sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nichtkopierbaren optischen Effekte gewinnen diese Pigmente auch zunehmende Bedeutung für die Herstellung von fälschungssicheren Wertschriften, wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Von besonderem Interesse sind goniochromatische Glanzpigmente, die einen winkelabhängigen Farbwechsel zwischen mehreren intensiven Interferenzfarben und damit ein attraktives Farbenspiel zeigen.

Bislang sind eine Reihe von goniochromatischen Glanzpigmenten auf metallischer Basis bekannt, die über physikalische Aufdampftechniken (US-A-3 438 796 und 5 135 812) oder durch Beschichtung von Metallplättchen mittels Gasphasenzersetzung flüchtiger Precursor (CVD = Chemical Vapor Deposition) oder durch naßchemische Beschichtung der Metallplättchen (EP-A-668 329 und EP-A-708 154) hergestellt werden.

Goniochromatische Glanzpigmente auf Basis transparenter, silikatischer Substrate oder beschichteter Eisen(III)oxidplättchen sind in der DE-A-196 18 569, der EP-A-753 545 und der älteren deutschen Patentanmeldung 198 08 657.1 beschrieben.

Aus der EP-A-832 943 ist die Umsetzung von mit reduziertem Titandioxid beschichteten Glimmerplättchen mit Aminoalkyl- und Alkoxygruppen enthaltenden Silanen zur Erhöhung der Schwitzwasserbeständigkeit bekannt.

Die bekannten Glanzpigmente unterscheiden sich von den erfindungsgemäßen Pigmenten durch die Art des Substratmaterials und/oder der aufgebrachten Beschichtungen.

Der Erfindung lag die Aufgabe zugrunde, weitere goniochromatische Glanzpigmente bereitzustellen, die sich durch vorteilhafte Anwendungseigenschaften auszeichnen und die koloristischen Möglichkeiten erweitern.

Demgemäß wurden die eingangs definierten goniochromatischen Glanzpigmente gefunden.

Weiterhin wurde ein Verfahren zur Herstellung dieser Glanzpigmente gefunden, welches dadurch gekennzeichnet ist, daß man die Beschichtungen (A) und (B) unabhängig voneinander entweder naßchemisch durch hydrolytische Zersetzung organischer oder anorganischer Metallverbindungen oder durch Gasphasenzersetzung flüchtiger, organischer oder anorganischer Metallverbindungen auf die in einer reduzierenden Atmosphäre erhitzten, titandioxidbeschichteten silikatischen Plättchen aufbringt.

Schließlich wurde die Verwendung der erfindungsgemäßen Glanzpigmente zur Einfärbung von Lacken, Druckfarben, Tinten, Kunststoffen, Gläsern, keramischen Produkten und Zubereitungen der dekorativen Kosmetik gefunden.

Die erfindungsgemäßen goniochromatischen Glanzpigmente basieren auf in einer reduzierenden Atmosphäre erhitzten, titandioxidbeschichteten silikatischen Plättchen, die eine Mehrfachbeschichtung aufweisen.

Als silikatische Plättchen sind dabei insbesondere helle oder weiße Glimmer geeignet, wobei Schuppen von vorzugsweise naß vermahlenem Muskovit besonders bevorzugt sind. Selbstverständlich können auch andere natürliche Glimmer, wie Phlogopit oder Biotit, künstliche Glimmer oder Talk- oder Glasschuppen als Ausgangsmaterial dienen.

Die silikatischen Plättchen sind mit einer im wesentlichen aus Titandioxid bestehenden Schicht belegt, die als untergeordnete Bestandteile (im allgemeinen < 5 Gew.-%) weitere, vorzugsweise farblose, Metalloxide wie Zinndioxid, Zirkondioxid, Aluminiumoxid und Siliciumdioxid enthalten kann.

Die Größe der Silikatplättchen ist an sich nicht kritisch und kann auf den jeweiligen Anwendungszweck abgestimmt werden. In der Regel haben die Plättchen mittlere größte Durchmesser von etwa 1 bis 200 µm, insbesondere etwa 5 bis 100 µm, und Dicken von etwa 0,1 bis 1 µm, insbesondere um etwa 0,5 µm. Ihre spezifische freie Oberfläche (BET) liegt üblicherweise bei 1 bis 15 m²/g, insbesondere bei 3 bis 12 m²/g.

Die Dicke der TiO₂-Schicht bestimmt die Reflexionsfarbe der Plättchen und beträgt vorzugsweise 50 bis 100 nm (Silber) oder 300 bis 340 nm (blau; optische Schichtdicken).

Bei den erfindungsgemäßen Glanzpigmenten sind die titandioxidbeschichteten Silikatplättchen in einer reduzierenden Gasatmosphäre erhitzt worden.

Als reduzierende Gase eignen sich dabei z.B. Ammoniakgas, Wasserstoff, flüchtige Kohlenwasserstoffe (insbesondere C₁-C₄-Alkane) und deren Gemische. Diese Gase werden vorzugsweise im Gemisch mit Inertgasen wie Stickstoff eingesetzt (vgl. EP-A-735 115 und die dort u.a. genannte EP-A-322 071).

Bevorzugte reduzierende Gase sind Ammoniakgas und Gemische von Ammoniakgas mit flüchtigen Kohlenwasserstoffen wie Methan, Ethan und/oder Propan, für die ein Volumenverhältnis von etwa 95:5 bis 70:30 zu empfehlen ist. Der Stickstoffanteil an der Gesamtgasmenge der jeweils besonders bevorzugten Reduktionsgas/Inertgas-Mischungen liegt vorzugsweise bei bis zu 90 Vol.-% bzw. bei 10 bis 60 Vol.-%.

Geeignete Reduktionstemperaturen betragen bei der Reduktion mit Ammoniakgas vorzugsweise 750 bis 850°C und bei der Umsetzung mit Ammoniakgas/Kohlenwasserstoff-Gemischen bevorzugt >800 bis 900°C.

Bei der Reduktion werden blaue, reduzierte Titanspezies mit Oxidationszahlen <4 bis 2 (niedere Titanoxide wie Ti₃O₅, Ti₂O₃ bis zu TiO, Titanoxynitride sowie Titannitrid) gebildet. Üblicherweise werden 5 bis 100 Gew.-% des ursprünglich vorhandenen Titandioxids reduziert.

Reduzierte titandioxidbeschichtete Glimmerpigmente sind im Handel unter dem Namen Paliocrom® erhältlich.

Die reduzierten, titandioxidbeschichteten silikatischen Plättchen sind hochbrechend. Ihr Brechungsindex n beträgt in der Regel ≥ 2,0, vorzugsweise ≥ 2,4. Sie sind für sichtbares Licht in Abhängigkeit von der betrachteten Wellenlänge im wesentlichen durchlässig bis nahezu undurchlässig.

Die erfindungsgemäßen Glanzpigmente weisen eine farblose niedrigbrechende Beschichtung (A) in Kombination mit einer farblosen hochbrechenden Beschichtung (B) auf. Sie können mehrere gleiche oder verschiedene Kombinationen (Schichtpakete) (A) + (B) enthalten, bevorzugt ist aber die Belegung mit nur einem Schichtpaket (A) + (B).

Die farblose niedrigbrechende Beschichtung (A) hat einen Brechungsindex n ≤ 1,8, vorzugsweise ≤ 1,6, und im sichtbaren Wellenlängenbereich eine Absorptionskonstante k = 0.

Als Schichtmaterial (A) eignen sich alle niedrigbrechenden farblosen Substanzen, die filmartig und dauerhaft auf die Substratplättchen aufgebracht werden können, wobei anorganische Materialien bevorzugt sind.

Besonders geeignet sind z.B. Metalloxide und Metalloxidhydrate wie Siliciumoxid, Siliciumoxidhydrat, Aluminiumoxid, Aluminiumoxidhydrat und deren Mischungen, wobei Siliciumoxid(hydrat) bevorzugt ist.

Die geometrische Schichtdicke der Beschichtung (A) beträgt im allgemeinen 50 bis 800 nm, vorzugsweise 100 bis 600 nm. Da die Schicht (A) im wesentlichen die Interferenzfarben der erfindungsgemäßen Pigmente bestimmt, hat sie für ein besonders ausgeprägtes Farbenspiel zeigende und daher auch bevorzugte Glanzpigmente, die nur ein Schichtpaket (A) + (B) aufweisen, eine Mindestschichtdicke von etwa 200 nm. Sind mehrere (z.B. 2, 3 oder 4) Schichtpakete (A) + (B) vorhanden, dann liegt die Schichtdicke von (A) bevorzugt bei 50 bis 200 nm.

Mit wachsender Schichtdicke von (A) sind beim trockenen Pigmentpulver in der Aufsicht mehrmals nacheinander die Interferenzfarben Blau-Grün-Gold-Rot-Violett zu beobachten, wobei beginnend mit dem Blau zweiter Ordnung die Winkelabhängigkeit des Farbtons zunimmt. Die Interferenzfarben sind jedoch nur im trockenen Zustand sichtbar und verschwinden im feuchten Zustand oder im Lack vollständig. Durch die zusätzliche Beschichtung mit (B) wird die optisch variable Schicht auch in Lacken sichtbar.

Die farblose hochbrechende Beschichtung (B) hat einen Brechungsindex n ≥ 2,0, insbesondere ≥ 2,4, und im sichtbaren Wellenlängenbereich eine Absorptionskonstante k = 0.

Als Schichtmaterial (B) sind alle hochbrechenden farblosen Substanzen, die filmartig und dauerhaft auf die mit (A) belegten Eisenoxidplättchen aufgebracht werden können, geeignet.

Besonders geeignet sind neben Metallsulfiden wie Zinksulfid auch hier vor allem Metalloxide und Metalloxidhydrate, z.B. Titandioxid, Titanoxidhydrat, Zirkoniumdioxid, Zirkoniumoxidhydrat, Zinndioxid, Zinnoxidhydrat, Zinkoxid, Zinkoxidhydrat und deren Mischungen, wobei Titandioxid und Titanoxidhydrat und deren Mischungen mit bis zu etwa 5 Gew. -% der anderen Metalloxide, insbesondere Zinndioxid, bevorzugt sind. Titandioxid kann auch zusammen mit niedrigbrechenden farblosen Metalloxiden verwendet werden, wenn der Brechungsindex dieser Mischungen ≥ 2,0 ist.

Die Beschichtung (B) hat vorzugsweise eine niedrigere Schichtdicke als die Beschichtung (A). Bevorzugte geometrische Schichtdicken liegen für die Beschichtung (B) bei etwa 5 bis 50 nm, insbesondere bei 10 bis 40 nm.

Die erfindungsgemäß bevorzugte, im wesentlichen aus Titandioxid bestehende Beschichtung (B) hat vorzugsweise eine optische Schichtdicke von ≤ 100 nm, ist also für sich genommen silbrig und zeigt noch keine Interferenzeffekte.

Die erfindungsgemäßen Glanzpigmente zeichnen sich durch gutes Deckvermögen, hohe Helligkeitswerte, intensive blaue Farbe mit ausgeprägter Goniochromatizität und "seidenweiches Aussehen" in applizierter Form sowie den gleichmäßigen, homogenen und filmartigen Aufbau ihrer interferenzfähigen Beschichtung aus und erweitern damit die Palette der bekannten Glanzpigmente auf vorteilhafte Weise.

Bei dem erfindungsgemäßen Verfahren zur Herstellung der Glanzpigmente werden die Beschichtungen (A) und (B) unabhängig voneinander entweder naßchemisch durch hydrolytische Zersetzung organischer oder anorganischer Metallverbindungen oder durch Gasphasenzersetzung (CVD) geeigneter flüchtiger Metallverbindungen aufgebrächt.

Selbstverständlich können beide Vorgehensweisen beliebig zur Herstellung der einzelnen Schichten kombiniert werden. Werden beide Beschichtungen naßchemisch aufgebracht, so erübrigt sich die Zwischentrocknung der mit (A) beschichteten Substratplättchen; wird das gleiche Reaktionsmedium verwendet, so kann ebenfalls die Zwischenisolierung entfallen. Entsprechend ist die Zwischenisolierung üblicherweise auch bei der Durchführung beider Beschichtungsschritte nach dem CVD-Verfahren nicht erforderlich.

Zur Erzeugung der Silicium- und/oder Aluminiumoxid(hydrat)schichten (A) sind der naßchemische und der CVD-Herstellungsweg gleichermaßen geeignet.

Bei der naßchemischen Variante kann vorteilhaft gemäß dem in der EP-A-668 329 beschriebenen Verfahren vorgegangen werden, bei dem organische Silicium- und/oder Aluminiumverbindungen, bei welchen die organischen Reste über Sauerstoffatome an die Metalle gebunden sind, in Gegenwart der Substratplättchen und eines organischen Lösungsmittels, in welchem die Metallverbindungen löslich sind und welches mit Wasser mischbar ist, hydrolysiert werden.

Die bevorzugte Ausführungsform besteht dabei in der Hydrolyse der Metallalkoholate (insbesondere Tetraethoxysilan und Aluminiumtriisopropanolat) in Gegenwart eines Alkohols (insbesondere Isopropanol oder Ethanol) und von wäßrigem Ammoniak als Katalysator.

Verfahrenstechnisch geht man gemäß dem in der EP-A-668 329 beschriebenen Verfahren vorzugsweise so vor, daß man Substratplättchen, Isopropanol, Wasser und Ammoniak vorlegt, diese Mischung unter Rühren auf 40 bis 80°C, insbesondere 60 bis 70°C, erhitzt und eine Lösung des Metallalkoholats in Isopropanol kontinuierlich zudosiert. Nach einer Nachrührzeit von meist etwa 1 bis 15 h kühlt man die Mischung auf Raumtemperatur ab und isoliert das beschichtete Pigment durch Abfiltrieren und Trocknen.

Siliciumoxid(hydrat)beschichtungen (A) können vorteilhaft auch ausgehend von Alkalimetallsilikaten, insbesondere von Natronwasserglas, erzeugt werden.

Man geht dabei zweckmäßigerweise so vor, daß man die Substratplättchen in Wasser suspendiert, die Suspension auf etwa 20 bis 100°C, bevorzugt 40 bis 80°C, erhitzt, mit einer Base (insbesondere einer Alkalimetallhydroxidlösung wie Kalilauge oder Natronlauge) einen pH-Wert von in der Regel 4 bis 9, vorzugsweise 6,5 bis 8,5, insbesondere etwa 7,5, einstellt und die Alkalimetallsilikatlösung unter gleichzeitiger Zugabe einer wäßrigen anorganischen Säure wie Salzsäure, insbesondere einer verdünnten Salzsäure, zur Konstanthaltung des pH-Wertes zudosiert. Gegebenenfalls rührt man wenige min bis zu 2 h nach.

Bei der CVD-Variante kann man gemäß dem in der EP-A-708 154 beschriebenen Verfahren vorgehen. Hierbei werden Silane, die mindestens einen Alkanoyloxyrest enthalten, in der Gasphase mit Wasserdampf und, wenn die Silane auch Alkyl- oder Phenylreste aufweisen, Sauerstoff in Gegenwart der verwirbelten Substratplättchen zersetzt.

Bevorzugte Silane weisen dabei Alkoxy- und Alkanoyloxyreste auf, besonders bevorzugt ist Di-tert.-butoxydiacetoxysilan.

Zur Durchführung der CVD-Variante empfiehlt sich wie allgemein für CVD-Verfahren üblich die Verwendung eines Wirbelschichtreaktors. Die Substratplättchen werden im Reaktor unter Fluidisierung (Verwirbelung) mit einem inerten Wirbelgas wie Stickstoff auf die gewünschte Reaktionstemperatur (in der Regel 100 bis 600°C, bevorzugt 150 bis 300°C) erhitzt, Silan und Wasserdampf (sowie gegebenenfalls Sauerstoff) werden dann mit Hilfe inerter Trägergasströme (vorteilhaft Teilströmen des Wirbelgases) aus vorgeschalteten Verdampfergefäßen über getrennte Düsen eingeleitet, wobei die Silankonzentration zweckmäßigerweise bei ≤ 5 Vol.-%, vorzugsweise bei ≤ 2 Vol.-%, bezogen auf die Gesamtgasmenge im Reaktor, gehalten wird. Die Wasserdampfmenge sollte mindestens der stöchiometrisch zur Hydrolyse des Silans erforderlichen Menge entsprechen, vorzuziehen ist jedoch die 10 bis 100fache Menge.

Auch die Abscheidung der Beschichtungen (B) ist sowohl auf dem CVD-Weg als auch naßchemisch möglich.

Für die CVD-Variante eignen sich als Ausgangsverbindungen vor allem Metallalkoholate, Metallhalogenide und Metallorganyle. Bevorzugt sind dabei solche Verbindungen, die bei Temperaturen bis zu 200°C einen ausreichend hohen Dampfdruck haben, um eine einfache Verdampfung möglichst ohne Zersetzung zu gewährleisten.

Bei den Alkoholaten kommen aromatische Alkoholate wie Phenolate und Benzylalkoholate sowie aliphatische, vor allem C₁-C₄-Alkoholate wie n-, iso- und tert.-Butanolate, bevorzugt Methanolate und Ethanolate und insbesondere n-und iso-Propanolate und auch deren Mischungen in Betracht.

Als Metallhalogenide sind die Chloride bevorzugt.

Metallorganyle können z.B. Metallalkyle, insbesondere solche mit bis zu 4 C-Atomen in der Alkylkette, Metallalkenyle, Metallaryle, Metallarylalkyle und Metallalkylalkenyle sein.

Als geeignete Ausgangsverbindungen seien beispielsweise genannt:
- Alkoholate wie Titantetraethanolat, Titantetra-n- und -isopropanolat und insbesondere Mischungen von Titantetraethanolat und Titantetraisopropanolat, vorzugsweise im Molverhältnis von etwa 1:1, die sich durch niedrige Verdampfungstemperaturen (um 120°C) sowie niedrige Zersetzungstemperaturen (Hydrolyse mit Wasserdampf bei etwa 200°C möglich) auszeichnen, sowie Zirkonium-n- und -isopropanolat;
- Halogenide wie Titantetrachlorid, Zirkoniumtetrachlorid und Zinntetrachlorid;
- Organyle wie Zinntetramethyl, Zinntetra-n-butyl und Zinkdiethyl.

Die Zersetzung dieser Metallverbindungen zu sich filmartig auf den mit (A) belegten Substratplättchen abscheidenden Metalloxidschichten erfolgt zweckmäßigerweise ebenfalls in einem Wirbelschichtreaktor, wobei im Fall der Alkoholate Wasserdampf und gewünschtenfalls Luft, im Fall der Halogenide Wasserdampf und im Fall der Organyle Sauerstoff als zusätzliches Reaktionsgas eingesetzt wird. Geeignete Zersetzungstemperaturen betragen in der Regel 100 bis 600°C, vorzugsweise 150 bis 300°C (Alkoholate), 150 bis 350°C (Halogenide) bzw. 300 bis 500°C (Organyle).

Analog zu den Metalloxid(hydrat)beschichtungen (A) können die Metalloxid(hydrat)schichten (B) ebenfalls naßchemisch durch Hydrolyse von Metallalkoholaten (z.B. von Titanethanolat) in alkoholischem Medium oder vorzugsweise durch Hydrolyse von anorganischen Metallsalzen, insbesondere von Halogeniden, bevorzugt von Chloriden, in wäßriger Suspension aufgebracht werden.

Zur Abscheidung der bevorzugten Titandioxidschichten (B) kann man vorteilhaft so vorgehen, daß man eine wäßrige Suspension der mit (A) belegten Substratplättchen auf üblicherweise 50 bis 100°C, vorzugsweise 70 bis 80°C, erhitzt, mit einer Base (insbesondere einer Alkalimetallhydroxidlösung wie Kalilauge oder Natronlauge) einen pH-Wert von in der Regel 0,5 bis 5, bevorzugt 1,5 bis 2,5, insbesondere etwa 2,2, einstellt und eine Titantetrachloridlösung unter gleichzeitiger Basenzugabe zur Konstanthaltung des pH-Wertes zudosiert.

Sowohl das aus der Gasphase als auch das naßchemisch abgeschiedene Titanoxid(hydrat) ist nur unvollständig kristallisiert. Die amorphen Anteile können durch Calcinierung des isolierten (und getrockneten) Pigments in kristalline Form, üblicherweise in die Anatasmodifikation, überführt werden. Dazu erhitzt man das Pigment im allgemeinen etwa 1 bis 4 h auf 400 bis 1000°C. Soll die Titandioxidbeschichtung (B) nach der Calcinierung in der Rutilmodifikation vorliegen, so empfiehlt es sich, das Titanoxid(hydrat) durch gleichzeitiges Abscheiden von Zinnoxid(hydrat) mit etwa 0,5 bis 10 Gew.-% Zinndioxid zu dotieren, welches die Bildung der Rutilmodifikation begünstigt.

Mit Hilfe des erfindungsgemäßen Herstellungsverfahrens können die mehrfach beschichteten Glanzpigmente in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und insbesondere von Lacken, Tinten und Druckfarben, auch Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen Glanzpigmenten auf der Basis von metalloxidbeschichteten Glimmer- und Metallpigmenten und bekannten goniochromatischen Glanzpigmenten verwenden.

### Beispiele

### Herstellung und Anwendung von erfindungsgemäßen Glanzpigmenten

Zur Beurteilung der Koloristik der erhaltenen Pigmente wurden jeweils 0,4 g Pigment in 3,6 g eines Polyester-Mischlacks mit 21 Gew.-% Feststoffanteil eingerührt und 2 min in einem Red Devil® dispergiert. Mit einer Rakel (200 µm Naßfilmdicke, einfache Rakelung) wurden anschließend Abzüge der pigmentierten Lacke auf einem schwarzweißen Karton angefertigt. Die Messung der CIELAB-Werte erfolgte nach dem Trocknen des Films mit einem Gonio-Spektralphotometer Multiflash (Fa. Optronik) bei einer Winkeldifferenz von 20° bis 115° zum Glanzwinkel über schwarzem Untergrund. Die Angaben der Farbwerte beziehen sich auf die Normlichtart D65. L entspricht der Helligkeit, a* dem Rot- bzw. Grünanteil und b* dem Blau- bzw. Gelbanteil, H ist der Farbwinkel und C das Chroma. Bei dieser Meßanordnung wird nur ein Teil des Farbenspiels, nämlich im wesentlichen die Farbe der Lackierung in Aufsicht, erfaßt.

### Beispiel

a) 100 g eines blausilbernen, mit Ammoniak reduzierten , TiO₂-Glimmerpigments (Paliocrom® Blausilber L6000, BASF) wurden in 1,5 1 Isopropanol aufgeschlämmt, zunächst mit 400 g Wasser und 40 g 25 gew.-%iger wäßriger Ammoniaklösung und nach Erhitzen auf 60°C in ca. 24 h mit einem Gemisch von 345 g Tetraethoxysilan und 345 g Isopropanol versetzt. Nach einer Nachrührzeit von ca. 2 h und Abkühlen der Suspension wurde das Produkt abfiltriert, mit Isopropanol gewaschen und unter vermindertem Druck bei 80°C getrocknet.
   Das getrocknete SiO₂-beschichtete Pigment (203 g) zeigte an der Luft eine violette, im Lack unsichtbare Körperfarbe.
b) Eine Suspension von 100g des SiO₂-beschichteten und getrockneten Produkts in 1350 ml Wasser wurde auf 75°C erhitzt und dann mit 32 gew.-%iger Salzsäure auf einen pH-Wert von 2,2 eingestellt. Dann wurden 120 ml einer wäßrigen Titantetrachloridlösung (200 g TiCl₄/l) in 120 min zugegeben. Durch gleichzeitige Zugabe von 32 gew.-%iger Matronlauge wurde der pH-Wert konstant bei 2,2 gehalten. Nach einer Nachrührzeit von 30 min und Abkühlen der Suspension wurde das Produkt abfiltriert, mit Wasser gewaschen und unter vermindertem Druck bei 80°C getrocknet.

Das erhaltene Pigment hatte einen Siliciumgehalt von 26,1 Gew.-% und einen Titangehalt von 12,9 Gew.-%. Im Lack appliziert, zeigte das Pigment gutes Deckvermögen und ein winkelabhängiges Farbenspiel von intensiv grünstichig Blau nach Violett auf hohem Helligkeitsniveau.

Farbmetrische Daten des erhaltenen Pigments:

| Meßwinkel in ° | L | a* | b* | C | H |
|---|---|---|---|---|---|
| 20 | 74,2 | -11,1 | -35,7 | 37,4 | 252,7 |
| 25 | 69,9 | -12,1 | -30,2 | 32,5 | 248,2 |
| 35 | 49,6 | -11,1 | -21,5 | 24,2 | 242,7 |
| 45 | 39,5 | -8,5 | -17,2 | 19,2 | 243,7. |
| 55 | 33,2 | -6,2 | -15,1 | 16,3 | 247,7 |
| 65 | 28,3 | -4,1 | -13,5 | 14,1 | 253,3 |
| 75 | 37,3 | -3,7 | -13,2 | 13,7 | 254,4 |
| 115 | 24,1 | -1,9 | -13,1 | 13,2 | 262,0 |

### Vergleichsbeispiel

Zum Vergleich wurden die farbmetrischen Daten des SiO2- und molybdänbeschichteten Glimmerpigments Paliocrom Blausilber L6000 aus Beispiel 4 der EP-A-753 545 bestimmt.

Dieses Pigment hatte einen Titangehalt von 7,7 Gew.-%, einen Siliciumgehalt von 29,6 Gew.-% und einen Molybdängehalt von 2,6 Gew.-%. Im Lack appliziert, zeigte dieses Pigment ebenfalls gutes Deckvermögen, jedoch nur ein winkelabhängiges Farbenspiel von grünstichig Blau nach Violett mit geringerer Farbstärke und auf deutlich niedrigerem Helligkeitsniveau.

Farbmetrische Daten des erhaltenen Pigments:

| Meßwinkel in ° | L | a* | b* | C | H |
|---|---|---|---|---|---|
| 20 | 58,2 | -8,8 | -22,3 | 23,9 | 248,5 |
| 25 | 48,7 | -9,1 | -18,6 | 20,7 | 244,0 |
| 35 | 33,3 | -8,2 | -12,3 | 14,8 | 236,1 |
| 45 | 23,0 | -6,6 | -8,4 | 10,7 | 231,7 |
| 55 | 16,4 | -4,9 | -6,4 | 8,1 | 232,3 |
| 65 | 11,5 | -3,0 | -5,4 | 6,1 | 241,0 |
| 75 | 10,6 | -2,6 | -5,1 | 5,7 | 242,9 |
| 115 | 7,2 | -0,8 | -3,9 | 4,0 | 259,0 |

## Patentansprüche

1. Goniochromatische Glanzpigmente auf der Basis von in einer reduzierenden Atmosphäre erhitzten, titandioxidbeschichteten silikatischen Plättchen, die mindestens ein Schichtpaket aus
A) einer farblosen Beschichtung mit einem Brechungsindex n ≤ 1,8 und
B) einer farblosen Beschichtung mit einem Brechungsindex n ≥ 2,0 aufweisen.

2. Glanzpigmente nach Anspruch 1, bei denen die Beschichtung (B) eine optische Schichtdicke von < 100 nm hat.

3. Glanzpigmente nach Anspruch 1 oder 2, bei denen die Beschichtung (A) im wesentlichen aus Siliciumoxid, Siliciumoxidhydrat, Aluminiumoxid und /oder Aluminiumoxidhydrat besteht.

4. Glanzpigmente nach den Ansprüchen 1 bis 3, bei denen die Beschichtung (B) im wesentlichen aus Titandioxid, Titanoxidhydrat, Zirkoniumdioxid, Zirkoniumoxidhydrat, Zinndioxid, Zinnoxidhydrat, Zinkoxid, Zinkoxidhydrat und/oder Zinksulfid besteht.

5. Glanzpigmente nach den Ansprüchen 1 bis 4, die nur ein Schichtpaket (A) + (B) enthalten.

6. Glanzpigmente nach den Ansprüchen 1 bis 5, bei denen die titandioxidbeschichteten silikatischen Plättchen in einer reduzierenden Atmosphäre, die Ammoniakgas, Wasserstoff oder flüchtige Kohlenwasserstoffe oder deren Gemische enthält, erhitzt worden sind.

7. Glanzpigmente nach den Ansprüchen 1 bis 6, bei denen die titandioxidbeschichteten silikatischen Plättchen in einer reduzierenden, Ammoniakgas oder ein Gemisch von Ammoniakgas und flüchtigen Kohlenwasserstoffen enthaltenden Atmosphäre erhitzt worden sind.

8. Verfahren zur Herstellung von Glanzpigmenten gemäß den Ansprüchen 1 bis 7, **dadurch gekennzeichnet, daß** man die Beschichtungen (A) und (B) unabhängig voneinander entweder naßchemisch durch hydrolytische Zersetzung organischer oder anorganischer Metallverbindungen oder durch Gasphasenzersetzung flüchtiger, organischer oder anorganischer Metallverbindungen auf die in einer reduzierenden Atmosphäre erhitzten, titandioxidbeschichteten silikatischen Plättchen aufbringt.

9. Verwendung von Glanzpigmenten gemäß den Ansprüchen 1 bis 7 zur Einfärbung von Lacken, Druckfarben, Tinten, Kunststoffen, Gläsern, keramischen Produkten und Zubereitungen der dekorativen Kosmetik.

## Claims

1. Goniochromatic luster pigments based on titanium dioxide-coated silicatic platelets which have been heated in a reducing atmosphere and which comprise at least one layer packet comprising
A) a colorless coating having a refractive index n ≤ 1.8 and
B) a colorless coating having a refractive index n ≥ 2.0.

2. Luster pigments as claimed in claim 1, wherein said coating (B) has an optical layer thickness of ≤ 100 nm.

3. Luster pigments as claimed in claim 1 or 2, wherein said coating (A) consists essentially of silicon oxide, silicon oxide hydrate, aluminum oxide and/or aluminum oxide hydrate.

4. Luster pigments as claimed in any of claims 1 to 3, wherein said coating (B) consists essentially of titanium dioxide, titanium oxide hydrate, zirconium dioxide, zirconium oxide hydrate, tin dioxide, tin oxide hydrate, zinc oxide, zinc oxide hydrate and/or zinc sulfide.

5. Luster pigments as claimed in any of claims 1 to 4, comprising just one layer packet (A) + (B).

6. Luster pigments as claimed in any of claims 1 to 5, wherefor the titanium dioxide-coated silicatic platelets have been heated in a reducing atmosphere comprising ammonia gas, hydrogen or volatile hydrocarbons or mixtures thereof.

7. Luster pigments as claimed in any of claims 1 to 6, wherefor the titanium dioxide-coated silicatic platelets have been heated in a reducing atmosphere comprising ammonia gas or a mixture of ammonia gas and volatile hydrocarbons.

8. A process for producing luster pigments as claimed in any of claims 1 to 7, which comprises applying said coatings (A) and (B) independently of each other to said titanium dioxide-coated silicatic platelets which have been heated in a reducing atmosphere either wet-chemically by hydrolytic decomposition of organic or inorganic metal compounds or by gas phase decomposition of volatile, organic or inorganic metal compounds.

9. The use of luster pigments as claimed in any of claims 1 to 7 for coloring coatings, inks, including printing inks, plastics, glasses, ceramic products and decorative cosmetic preparations.

## Revendications

1. Pigments brillants goniochromatiques sur la base de plaquettes silicatées revêtues de dioxyde de titane, chauffées dans une atmosphère réductrice, qui présentent au moins un paquet de couches constitué
A)d'un revêtement incolore ayant un indice de réfraction n ≤ 1,8 et
B)d'un revêtement incolore ayant un indice de réfraction n ≥ 2,0.

2. Pigments brillants selon la revendication 1, dans lesquels le revêtement (B) a une épaisseur optique de couche de ≤ 100 nm.

3. Pigments brillants selon la revendication 1 ou 2, dans lesquels le revêtement (A) est constitué pour l'essentiel d'oxyde de silicium, d'oxyde de silicium hydraté, d'oxyde d'aluminium et/ou d'oxyde d'aluminium hydraté.

4. Pigments brillants selon les revendications 1 à 3, dans lesquels le revêtement (B) est constitué pour l'essentiel de dioxyde de titane, d'oxyde de titane hydraté, de dioxyde de zirconium, d'oxyde de zirconium hydraté, de dioxyde d'étain, d'oxyde d'étain hydraté, d'oxyde de zinc, d'oxyde de zinc hydraté et/ou de sulfure de zinc.

5. Pigments brillants selon les revendications 1 à 4, qui ne contiennent qu'un paquet de couches (A) + (B).

6. Pigments brillants selon les revendications 1 à 5, dans lesquels les plaquettes silicatées revêtues de dioxyde de titane ont été chauffées dans une atmosphère réductrice, qui contient de l'ammoniac gazeux, de l'hydrogène ou des hydrocarbures volatiles ou leurs mélanges.

7. Pigments brillants selon les revendications 1 à 6, dans lesquels les plaquettes silicatées revêtues de dioxyde de titane ont été chauffées dans une atmosphère réductrice contenant de l'ammoniac gazeux ou un mélange d'ammoniac gazeux et d'hydrocarbures volatiles.

8. Procédé de fabrication de pigments brillants selon les revendications 1 à 7, **caractérisé en ce que** l'on dépose les revêtements (A) et (B) indépendamment l'un de l'autre soit de manière chimique par voie humide par décomposition hydrolytique de combinaisons métalliques organiques ou inorganiques soit par décomposition en phase gazeuse de combinaisons métalliques organiques ou inorganiques volatiles, sur les plaquettes silicatées revêtues de dioxyde de titane, chauffées dans une atmosphère réductrice.

9. Utilisation de pigments brillants selon les revendications 1 à 7 pour la coloration de laques, d'encres d'imprimerie, d'encres, de matières plastiques, de verres, de produits céramiques et de préparations de l'esthétique décorative.
